# EUROPÄISCHE PATENTANMELDUNG

(11) **EP 3 992 172 A1**
(43) Veröffentlichungstag der Anmeldung: **04.05.2022**
(21) Anmeldenummer: 20204783.3
(22) Anmeldetag: 29.10.2020
(51) Int. Cl.: C07C 5/333, C07C 11/06, C10G 9/00

(54) **VERFAHREN UND ANLAGE ZUR HERSTELLUNG VON OLEFINEN**

(71) Anmelder: Linde GmbH, 82049 Pullach (DE)
(72) Erfinder: Höfel, Torben, 81543 München (DE); Fritz, Helmut, 81375 München (DE)
(74) Vertreter: DehnsGermany Partnerschaft von Patentanwälten

(57) **Zusammenfassung**

Es wird ein Verfahren (100) zur Herstellung von Olefinen vorgeschlagen, bei dem ein Propan und Wasserstoff enthaltender erster Einsatzstrom unter Erhalt eines zumindest Propylen, Propan, Ethan und/oder Ethylen, Methan und Wasserstoff enthaltenden ersten Produktstroms einer Propandehydrierung (10) unterworfen wird, und bei dem zumindest einen Teil des ersten Produktstroms einer ersten Trennsequenz (11, 12) unterworfen wird. Hierbei ist vorgesehen, dass ein zweiter Einsatzstrom unter Erhalt eines zweiten Produktstroms einer Dampfspaltung (20) unterworfen wird, dass zumindest ein Teil des zweiten Produktstroms einer Rohgasverdichtung (21) und danach einer zweiten Trennsequenz (22) unterworfen wird, dass in der ersten Trennsequenz (12) eine zumindest Ethan und/oder Ethylen enthaltende Transferfraktion gebildet wird, und dass zumindest ein Teil der Transferfraktion in die Dampfspaltung (20) oder die Rohgasverdichtung (21) überführt wird. Eine entsprechende Anlage ist ebenfalls Gegenstand der Erfindung.

## Beschreibung

Die Erfindung betrifft ein Verfahren und eine Anlage zur Herstellung von Olefinen gemäß den jeweiligen Oberbegriffen der unabhängigen Patentansprüche.

### Hintergrund der Erfindung

Die Herstellung von Propylen aus Propan durch Dehydrierung (kurz Propandehydrierung, engl. Propane Dehydrogenation, PDH) ist bekannt und stellt ein kommerziell verfügbares und etabliertes Verfahren der chemischen Industrie dar. Für eine Übersicht sei beispielsweise auf den Artikel "Propene" in Ullmann's Encyclopedia of Industrial Chemistry, 2013, DOI: 10.1002/14356007.a22_211.pub3, insbesondere Kapitel 3.3.1, "Propane Dehydrogenation", verwiesen.

Die Propandehydrierung ist typischerweise durch eine sehr hohe Selektivität zum Hauptprodukt Propylen charakterisiert. Wesentliche weitere Komponenten im Produktgas sind nicht umgesetztes Propan und Wasserstoff. Die kohlenstoffhaltigen Nebenkomponenten sind überwiegend Ethan und/oder Ethylen und Methan und Kohlenwasserstoffe mit vier und mehr Kohlenstoffatomen.

Es wäre wünschenswert, zumindest einen Teil der erwähnten Nebenkomponenten in wirtschaftlich vorteilhafter Weise nutzen zu können.

### Offenbarung der Erfindung

Diese Aufgabe wird durch ein Verfahren und eine Anlage zur Herstellung von Olefinen gemäß den jeweiligen Oberbegriffen der unabhängigen Patentansprüche gelöst. Ausgestaltungen der Erfindung sind Gegenstand der jeweiligen abhängigen Patentansprüche und der nachfolgenden Beschreibung.

In dem erfindungsgemäß vorgeschlagenen Verfahren zur Herstellung von Olefinen wird ein Propan und Wasserstoff enthaltender erster Einsatzstrom unter Erhalt eines zumindest Propylen, Propan, Ethan und/oder Ethylen, Methan und Wasserstoff enthaltenden ersten Produktstroms einer Propandehydrierung unterworfen, und zumindest einen Teil des ersten Produktstroms wird einer ersten Trennsequenz unterworfen. Erfindungsgemäß wird ein zweiter Einsatzstrom unter Erhalt eines zweiten Produktstroms einer Dampfspaltung unterworfen und zumindest ein Teil des zweiten Produktstroms wird einer Rohgasverdichtung und danach einer zweiten Trennsequenz unterworfen. In der ersten Trennsequenz wird erfindungsgemäß eine zumindest Ethan und/oder Ethylen enthaltende Transferfraktion gebildet, und zumindest ein Teil der Transferfraktion wird in die Dampfspaltung oder die Rohgasverdichtung überführt.

Je nach Verfahrensvariante kann in der Propandehydrierung und ggf. nachgeschaltete Schritte wie unten erläutert ein Gasgemisch gebildet werden, das deutlich mehr Ethan als Ethylen enthält oder umgekehrt, was hier durch die allgemeine Formulierung ausgedrückt wird, dass das Gasgemisch "Ethan und/oder Ethylen enthält". Die vorliegende Erfindung eignet sich für beide Varianten. Im Fall eines Gasgemischs mit mehr Ethan als Ethylen kann eine Transferfraktion mit entsprechenden Gehalten insbesondere in die Dampfspaltung, im Fall eines Gasgemischs mit mehr Ethylen als Ethan insbesondere in die nachgeschaltete Rohgasverdichtung geführt werden.

Die vorliegende Erfindung schafft auf diese Weise ein Verfahren, das die Einsparung von frischem Einsatzstoff zur Dampfspaltung ohne zusätzlichen apparativen Aufwand. ermöglicht. Die Erfindung kann insbesondere dann Anwendung finden, wenn eine Propandehydrierung sowie eine Dampfspaltung in örtlicher Nähe zueinander geplant werden bzw. mittels entsprechender Anlagenkomponenten realisiert werden sollen.

Bei einigen Verfahren zur Propandehydrierung (beispielsweise dem sog. Oleflex-Verfahren von UOP) erfolgt zunächst die Generierung einer Gasfraktion und einer Flüssigfraktion. Die Gasfraktion enthält dabei den überwiegenden Teil des Wasserstoffs aus dem Produktstrom der Propandehydrierung (hier als "erster" Produktstrom bezeichnet). Sie enthält außerdem einen großen Teil des Methans aus dem Produktstrom der Propandehydrierung sowie geringe Mengen an Kohlenwasserstoffen mit zwei und drei Kohlenstoffatomen. Die Flüssigfraktion enthält wiederum den überwiegenden Teil der Kohlenwasserstoffe mit zwei und drei Kohlenstoffatomen und nur geringe Mengen des Wasserstoffs und Methans. Ferner kann eine Schwerfraktion mit Kohlenwasserstoffen mit vier und mehr Kohlenstoffatomen gebildet werden.

Die vorliegende Erfindung sieht daher in einer entsprechenden Ausgestaltung vor, dass die erste Trennsequenz einen ersten Trennschritt umfasst, dem zumindest ein Teil des ersten Produktstroms zugeführt wird, und in dem eine an Wasserstoff und Methan angereicherte Gasfraktion und eine an Wasserstoff und Methan abgereicherte Flüssigfraktion gebildet wird, und bei dem die erste Trennsequenz einen zweiten Trennschritt umfasst, dem zumindest ein Teil der Flüssigfraktion zugeführt wird, und in dem die Transferfraktion gebildet wird. Die Begriffe "angereichert" und "abgereichert" beziehen sich dabei auf einen jeweiligen Gehalt in dem ersten Produktstrom, wobei bei einem Gehalt von mehr als dem 1,5-fachen, 2-fachen, 5-fachen oder 10-fachen von einer "Anreicherung" und einem Gehalt von weniger als dem 0,5-fachen, 0,25-fachen, oder 0,1-fachen von einer "Abreicherung" gesprochen wird.

In einem Folgeschritt erfolgt in den erläuterten Verfahren die Trennung der Flüssigfraktion, typischerweise bei einem Druck zwischen 20 und 35 bar (abs.) zwischen Kohlenwasserstoffen mit zwei und drei Kohlenstoffatomen. Entsprechend liegt dann ein gasförmiges Produkt mit Kohlenwasserstoffen mit zwei Kohlenwasserstoffen und ggf. leichteren Komponenten vor, welches nur geringe Mengen Methan, Wasserstoff sowie möglicherweise Spuren von Kohlenmonoxid, Kohlendioxid und Acetylen enthält. Je nach der Ausgestaltung der Propandehydrierung besteht der Anteil an Kohlenwasserstoffen mit zwei Kohlenstoffatomen, wie erwähnt, entweder überwiegend aus Ethan oder aus Ehylen. Der Begriff "reich" bzw. "überwiegend" soll dabei hier für einen Gehalt von mehr als 50%, 60%, 70%, 80% oder 90% stehen, und der Begriff "geringe Mengen" für einen Gehalt von weniger als 10%, 5% oder 1%, wobei die Prozentangaben hier jeweils Molanteile bezeichnen sollen, falls nicht anders angegeben.

In dem erfindungsgemäßen Verfahren wird in einer entsprechenden Ausgestaltung also der zweite Trennschritt auf einem Druckniveau von 20 bis 35 bar (abs.) durchgeführt. Die Transferfraktion kann in dem zweiten Trennschritt derart gebildet werden, dass sie 0,01 bis 1,5% Wasserstoff, 0 bis 0,1% Kohlenmonoxid, 0 bis 0,015% Kohlendioxid, 5 bis 25% Methan, 0 bis 0,1% Acetylen, 60 bis 90% Ethan und/oder Ethylen und 0,05 bis 0,15% Kohlenwasserstoffe mit drei Kohlenstoffatomen enthält. In einem spezifischen Beispiel sind 1% Wasserstoff, 0,07% Kohlenmonoxid, 0,01% Kohlendioxid, 16% Methan, 0,02% Acetylen, 7% Ethylen, 76% Ethan und 0,1% Kohlenwasserstoffe mit drei Kohlenstoffatomen enthalten. Dieses spezifische Beispiel betrifft also den Fall, dass in der Propandehydrierung ein Gasgemisch mit mehr Ethan als Ethylen gebildet wird und daher auch die Transferfraktion einen höheren Ethan- als Ethylengehalt aufweist.

Wie erwähnt, kann das Verfahren im Rahmen der vorliegenden Erfindung in Form zweier Verfahrensalternativen realisiert werden, d.h. Zuführung des Transferstroms in die Dampfspaltung ("Variante 1") und in die der Dampfspaltung nachgeschaltete und der Trennsequenz vorgeschaltete Rohgasverdichtung ("Variante 2"). Die Wahl der jeweiligen Verfahrensalternativen richtet sich, wie angesprochen, insbesondere nach dem Ethangehalt in der Transferfraktion.

Aufgrund des typischerweise hohen Druckes, auf dem der vorteilhafterweise mehr Ethan als Ethylen aufweisende Transferstrom gebildet wird, kann dieser in Variante 1 ohne weitere Verdichtung direkt zu einem Spaltofen geführt werden (ggf. über eine Vorwärmung), da die zur Spaltung vorgesehene Komponente Ethan in dem Transferstrom bereits in hoher Konzentration vorliegt. Außerdem ist davon auszugehen, dass keine der anderen Komponenten in der zu erwartenden Konzentration den Spaltprozess wesentlich negativ beeinflusst. Es ist zudem anzunehmen, dass das enthaltene Ethylen zu wesentlichen Teilen den Spaltprozess übersteht und ebenfalls als Produkt gewonnen werden kann. Diese Variante hat kaum eine zusätzliche Belastung des Trennteils zur Folge und es kann anderer Einsatzstoff zum Spaltprozess in entsprechender Menge eingespart werden. Diese Variante ist also besonders bevorzugt, wenn der Transferstrom mehr Ethan als Ethylen enthält, wie beispielsweise im spezifischen oben erwähnten Beispiel.

Mit Variante 2 kann ausgeschlossen werden, dass der Spaltprozess durch andere Komponenten negativ beeinflusst wird. Variante 2 eignet sich insbesondere für die erwähnten Fälle, in denen der Transferstrom mehr Ethylen als Ethan enthält. Der Strom wird zur Rohgasverdichtung geführt und zwar insbesondere vor die Laugewäsche, die hier definitionsgemäß Teil der zweiten Trennsequenz sein soll. Hiermit wird gewährleistet, dass ggf. noch geringe Spuren von beispielsweise Kohlendioxid entfernt werden. Zusammen mit dem Spaltgas wird der Transferstrom hier in der Verdichtung nach der Laugewäsche auf den Rohgasverdichterenddruck verdichtet. Acetylene aus dem Transferstrom (sofern in Spuren enthalten) werden in der folgenden Hydrierung hydriert. Methan und ggf. leichtere Komponenten werden in einem entsprechenden Trennschritt in der ersten Trennsequenz, Kohlenwasserstoffe mit drei und mehr Kohlenstoffatomen in einem nachgeordneten Trennschritt entfernt. Ethylen kann in einem sog. C2-Splitter gewonnen werden. Hierbei spielt es keine Rolle, in welcher Reihenfolge die Hydrierung und die genannten Trennschritte erfolgen. Abgetrenntes Ethan, das aus dem Transferstrom und aus dem zweiten Produktstrom stammt, kann zum Spaltprozess zurückgeführt werden, so dass auch auf diese Weise eine entsprechende Menge an Einsatz eingespart wird.

In anderen als den zuvor erläuterten Technologien erfolgt nicht unbedingt die Vortrennung des ersten Produktstroms in eine Gasfraktion und eine Flüssigfraktion. Stattdessen erfolgt hier direkt eine Trennung des gesamten ersten Produktstroms zwischen zwei und drei Kohlenstoffatomen. In diesem Falle wäre es nicht sinnvoll, eine entsprechend erhaltene leichtere Fraktion direkt wie zuvor beschrieben zum Dampfspalten zu fahren, da das Kopfprodukt auch sehr reich an Wasserstoff und Methan sein kann. Für den Fall, dass dieses Kopfprodukt zunächst zur Wasserstoffgewinnung in einer Druckwechseladsorption genutzt wird, kann man das an Ethan und/oder Ethylen angereicherte Tailgas der Druckwechseladsorption jedoch vorteilhafterweise zur Rohgasverdichtung stromab der Dampfspaltung zu führen. Da das Tailgas der Druckwechseladsorption einen geringen Druck von typischerweise 1,2 bis 8 bar (abs.) aufweist, ist eine Einspeisung direkt in die Dampfspaltung weniger vorteilhaft.

In den erläuterten Ausgestaltungen wird in der der ersten Trennsequenz also eine wasserstoff- und methanreiche Fraktion gebildet, von der zumindest ein Teil in unveränderter Zusammensetzung als die Transferfraktion verwendet wird, oder aus zumindest einem Teil derer die Transferfraktion gebildet wird, insbesondere unter Verwendung einer Druckwechseladsorption.

Bei geeignetem Druck wird in allen Ausgestaltungen die Transferfraktion ohne weitere Verdichtung der Dampfspaltung zugeführt, so dass sich zusätzliche Verdichtungseinrichtungen einsparen lassen.

Die Erfindung erstreckt sich auch auf eine Anlage zur Herstellung von Olefinen, die dafür eingerichtet ist, einen Propan und Wasserstoff enthaltenden ersten Einsatzstrom unter Erhalt eines zumindest Propylen, Propan, Ethan und/oder Ethylen, Methan und Wasserstoff enthaltenden ersten Produktstroms einer Propandehydrierung zu unterwerfen, und die dafür eingerichtet ist, zumindest einen Teil des ersten Produktstroms einer ersten Trennsequenz zu unterwerfen. Erfindungsgemäß weist die Anlage Mittel auf, die dafür eingerichtet sind, einen zweiten Einsatzstrom unter Erhalt eines zweiten Produktstroms einer Dampfspaltung zu unterwerfen, zumindest einen Teil des zweiten Produktstroms einer Rohgasverdichtung und danach einer zweiten Trennsequenz zu unterwerfen, in der ersten Trennsequenz eine zumindest Ethan und/oder Ethylen enthaltende Transferfraktion zu bilden, und zumindest einen Teil der Transferfraktion in die Dampfspaltung oder die Rohgasverdichtung zu überführen.

Zu der erfindungsgemäß bereitgestellten Anlage und ihren Merkmalen sei auf den auf die obigen Erläuterungen bezüglich des erfindungsgemäßen Verfahrens ausdrücklich verwiesen, da diese eine entsprechende Anlage in gleicher Weise betreffen. Entsprechendes gilt insbesondere für eine Ausgestaltung einer entsprechenden Anlage, die vorteilhafterweise zur Ausführung eines entsprechenden Verfahrens in einer beliebigen Ausgestaltung eingerichtet ist.

Die Erfindung wird nachfolgend unter Bezugnahme auf die Figur, die eine Ausgestaltung der vorliegenden Erfindung veranschaulicht, weiter erläutert.

### Figurenbeschreibung

Wenn nachfolgend auf Verfahrensschritte Bezug genommen wird, betreffen die entsprechenden Erläuterungen in gleicher Weise Anlagenkomponenten, mit denen diese Verfahrensschritte durchgeführt werden, und umgekehrt.

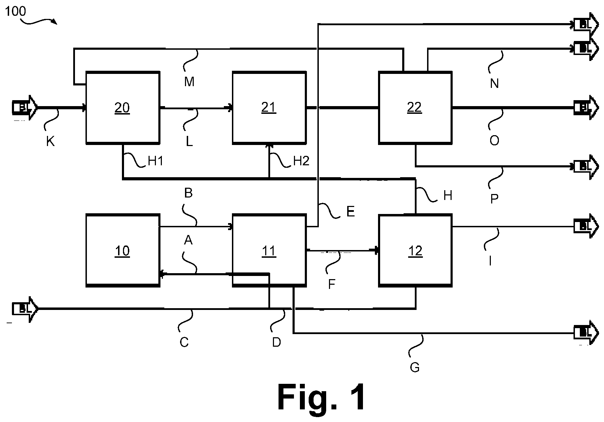

In Figur 1 ist ein Verfahren 100 zur Herstellung von Olefinen gemäß einer Ausgestaltung der vorliegenden Erfindung veranschaulicht.

In dem in Figur 1 veranschaulichten Verfahren 100 wird ein Propan und Wasserstoff enthaltender erster Einsatzstrom A unter Erhalt eines zumindest Propylen, Propan, Ethan, Methan und Wasserstoff enthaltenden ersten Produktstroms B einer Propandehydrierung 10 unterworfen. Zumindest ein Teil des ersten Produktstroms B wird einer einen ersten und einen zweiten Trennschritt umfassenden ersten Trennsequenz 11, 12 unterworfen.

In dem ersten Trennschritt 11 werden dabei eine an Wasserstoff und Methan angereicherte Gasfraktion und eine an Wasserstoff und Methan abgereicherte Flüssigfraktion gebildet. Der Wasserstoff der Gasfraktion wird zu einem Teil mit einem Propaneinsatz C und einem Propanrecycle D zu dem ersten Einsatzstrom A vereinigt. Ein weiterer Teil wird in Form eines Stoffstroms E zur Anlagengrenze (in Figur 1 durchgängig mit BL bezeichnet) geführt. Die Flüssigfraktion wird in Form eines Stoffstroms F dem zweiten Trennschritt 12 zugeführt. In dem ersten Trennschritt 11 wird ferner eine Fraktion mit Kohlenwasserstoffen mit vier und mehr Kohlenstoffatomen gebildet und in Form eines Stoffstroms G zur Anlagengrenze BL geführt.

Die Flüssigfraktion enthält insbesondere Ethan und/oder Ethylen, Propan und Propylen sowie nicht in die in dem ersten Trennschritt 11 abgetrennte Gasfraktion übergegangene leichtere Komponenten. In dem zweiten Trennschritt wird dabei eine Transferfraktion gebildet, die Ethan und/oder Ethylen und ggf. die leichteren Komponenten enthält, und die in Form eines Stoffstroms H aus dem zweiten Trennschritt 12 ausgeführt wird. Ferner wird eine in Form eines Stoffstroms I zur Anlagengrenze BL geführte Propylenproduktfraktion aus dem zweiten Trennschritt 12 ausgeführt, ebenso wie eine Propanfraktion, die in Form des bereits zuvor erwähnten Recyclestroms D bereitgestellt wird.

Ein zweiter Einsatzstrom K wird unter Erhalt eines zweiten Produktstroms L einer Dampfspaltung 20 unterworfen. Zumindest ein Teil des zweiten Produktstroms L einer Rohgasverdichtung 21 und danach einer zweiten Trennsequenz 22 unterworfen, wobei letztere hier in Form eines einzigen Funktionsblocks veranschaulicht ist, aber unterschiedliche Trenn- und Aufbereitungsschritte umfassen kann. Die Transferfraktion H wird, wie hier in Form von Alternativströmen H1 und H2 veranschaulicht, in die Dampfspaltung 20 oder die Rohgasverdichtung 21 überführt.

In der zweiten Trennsequenz werden in der hier dargestellten beispielhaften Ausgestaltung eine Paraffinfraktion, die in Form eines Recyclestroms M in die Dampfspaltung 20 zurückgeführt wird, eine Tailgasfraktion, die in Form eines Stoffstroms N zur Anlagengrenze BL geführt wird, eine Ethylenproduktfraktion, die in Form eines Stoffstroms O zur Anlagengrenze BL geführt wird, und eine Fraktion mit Kohlenwasserstoffen mit drei und mehr Kohlenstoffatomen, die in Form eines Stoffstroms P zur Anlagengrenze BL geführt wird, gebildet.

## Patentansprüche

1. Verfahren (100) zur Herstellung von Olefinen, bei dem ein Propan und Wasserstoff enthaltender erster Einsatzstrom unter Erhalt eines zumindest Propylen, Propan, Ethan und/oder Ethylen, Methan und Wasserstoff enthaltenden ersten Produktstroms einer Propandehydrierung (10) unterworfen wird, und bei dem zumindest einen Teil des ersten Produktstroms einer ersten Trennsequenz (11, 12) unterworfen wird, **dadurch gekennzeichnet, dass** ein zweiter Einsatzstrom unter Erhalt eines zweiten Produktstroms einer Dampfspaltung (20) unterworfen wird, dass zumindest ein Teil des zweiten Produktstroms einer Rohgasverdichtung (21) und danach einer zweiten Trennsequenz (22) unterworfen wird, dass in der ersten Trennsequenz (12) eine zumindest Ethan und/oder Ethylen enthaltende Transferfraktion gebildet wird, und dass zumindest ein Teil der Transferfraktion in die Dampfspaltung (20) oder die Rohgasverdichtung (21) überführt wird.

2. Verfahren (100) nach Anspruch 1, bei dem die erste Trennsequenz (11, 12) einen ersten Trennschritt (11) umfasst, dem zumindest ein Teil des ersten Produktstroms zugeführt wird, und in dem eine an Wasserstoff und Methan angereicherte Gasfraktion und eine an Wasserstoff und Methan abgereicherte Flüssigfraktion gebildet wird, und bei dem die erste Trennsequenz (11, 12) einen zweiten Trennschritt (12) umfasst, dem zumindest ein Teil der Flüssigfraktion zugeführt wird, und in dem die Transferfraktion gebildet wird.

3. Verfahren (100) nach Anspruch 2, bei dem der zweite Trennschritt (12) auf einem Druckniveau von 20 bis 35 bar (abs.) durchgeführt wird.

4. Verfahren (100) nach Anspruch 2 oder 3, bei dem die Transferfraktion 0,01 bis 1,5% Wasserstoff, 0 bis 0,1% Kohlenmonoxid, 0 bis 0,015% Kohlendioxid, 5 bis 25% Methan, 0 bis 0,1% Acetylen, 60 bis 90% Ethan und/oder Ethylen und 0,05 bis 0,15% Kohlenwasserstoffe mit drei Kohlenstoffatomen enthält.

5. Verfahren (100) nach Anspruch 1, bei dem in der ersten Trennsequenz eine wasserstoff- und methanreiche sowie Ethan und/oder Ethylen enthaltende Fraktion gebildet wird, von der zumindest ein Teil in unveränderter Zusammensetzung als die Transferfraktion verwendet wird, oder aus zumindest einem Teil derer die Transferfraktion gebildet wird.

6. Verfahren (100) nach Anspruch 5, bei dem die Transferfraktion unter Verwendung einer Druckwechseladsorption aus zumindest einem Teil der wasserstoff- und methanreichen sowie Ethan und/oder Ethylen enthaltenden Fraktion gebildet wird.

7. Verfahren (100) nach einem der vorstehenden Ansprüche, bei dem die Transferfraktion ohne weitere Verdichtung der Dampfspaltung (20) zugeführt wird.

8. Anlage zur Herstellung von Olefinen, die dafür eingerichtet ist, einen Propan und Wasserstoff enthaltenden ersten Einsatzstrom unter Erhalt eines zumindest Propylen, Propan, Ethan und/oder Ethylen, Methan und Wasserstoff enthaltenden ersten Produktstroms einer Propandehydrierung (10) zu unterwerfen, und die dafür eingerichtet ist, zumindest einen Teil des ersten Produktstroms einer ersten Trennsequenz (11, 12) zu unterwerfen, **dadurch gekennzeichnet, dass** die Anlage Mittel aufweist, die dafür eingerichtet sind, einen zweiten Einsatzstrom unter Erhalt eines zweiten Produktstroms einer Dampfspaltung (20) zu unterwerfen, zumindest einen Teil des zweiten Produktstroms einer Rohgasverdichtung (21) und danach einer zweiten Trennsequenz (22) zu unterwerfen, in der ersten Trennsequenz (12) eine zumindest Ethan und/oder Ethylen enthaltende Transferfraktion zu bilden, und zumindest einen Teil der Transferfraktion in die Dampfspaltung (20) oder die Rohgasverdichtung (21) zu überführen.

9. Anlage nach Anspruch 8, die zur Durchführung eines Verfahrens nach einem der Ansprüche 1 bis 7 eingerichtet ist.
